# FASCICULE DE BREVET EUROPEEN

(11) **EP 3 559 635 B1**
(45) Date de publication et mention de la délivrance du brevet: **03.11.2021**
(21) Numéro de dépôt: 17832523.9
(22) Date de dépôt: 21.12.2017
(51) Int. Cl.: G01N 21/47, G01N 21/51, G01N 15/06, G01N 15/14, C12Q 1/02, G01N 15/00, G01N 15/10

(54) **DISPOSITIF ET PROCÉDÉ POUR OBSERVER LE RAYONNEMENT RÉTRODIFFUSÉ PAR UN OBJET**
VORRICHTUNG UND VERFAHREN ZUR BEOBACHTUNG DER VON EINEM OBJEKT RÜCKGESTREUTEN STRAHLUNG
DEVICE AND METHOD FOR OBSERVING THE RADIATION BACKSCATTERED BY AN OBJECT

(30) Priorité: 26.12.2016 FR 1663396
(43) Date de publication de la demande: 30.10.2019
(73) Titulaire: Commissariat à l'Energie Atomique et aux Energies Alternatives, 75015 Paris (FR); Intelligence Artificielle Applications, 34060 Montpellier Cedex 2 (FR)
(72) Inventeur: SCHULTZ, Emmanuelle, 38120 Saint Egreve (FR); DECQ, Damien, 38000 Grenoble (FR); ROCH, Michel, 34670 Saint Bres (FR); BENAHMED, Selimen, 38054 Grenoble cedex 09 (FR)
(74) Mandataire: Le Goaller, Christophe
(86) Numéro de dépôt international: PCT/FR2017/053768
(87) Numéro de publication internationale: WO 2018/122504

(56) Documents cités:
- EP-A1- 3 054 281
- WO-A2-2016/054408
- US-A- 5 241 369
- US-A- 5 912 741
- US-A1- 2007 146 703
- KIM HUISUNG ET AL: "Reflected scatterometry for noninvasive interrogation of bacterial colonies", INTERNATIONAL SOCIETY FOR OPTICAL ENGINEERING, SPIE, PO BOX 10 BELLINGHAM WA 98227-0010 USA, vol. 21, no. 10, octobre 2016 (2016-10), page 107004, XP060075145, ISSN: 1083-3668, DOI: 10.1117/1.JBO.21.10.107004 [extrait le 2016-10-24] cité dans la demande
- E. Bae ET AL: "Label-free light-scattering sensors for high throughput screening of microbes in food" In: "High Throughput Screening for Food Safety Assessment", 2015, Elsevier, XP055404709, ISBN: 978-0-85709-801-6 pages 149-163, DOI: 10.1016/B978-0-85709-801-6.00006-X, le document en entier

## Description

### DOMAINE TECHNIQUE

Le domaine technique de l'invention est l'observation et l'identification d'un objet, notamment un objet biologique, en particulier une colonie bactérienne, sur la base d'une image d'un rayonnement rétrodiffusé par l'objet.

### ART ANTERIEUR

L'identification de microorganismes, et en particulier des bactéries, est un besoin concernant différents domaines. Dans le domaine du diagnostic, par exemple, l'identification de bactéries permet de connaître la nature d'agents pathogènes à l'origine d'une infection, et d'optimiser une prise en charge d'un patient. Par ailleurs, l'identification bactérienne est une technique de base dans l'épidémiologie, ou dans la lutte contre les infections nosocomiales. Au-delà de la santé, les applications peuvent concerner, de façon non exhaustive, l'hygiène, la sécurité, l'agroalimentaire.

On dispose actuellement d'une instrumentation performante et variée, permettant une telle identification. Les méthodes mises en œuvre sont notamment la spectrométrie de masse, la spectrométrie Raman, des tests colorimétriques, l'analyse morphologique de colonies, ou des techniques d'amplification d'acides nucléiques. Les méthodes mettant en œuvre une technique spectrométrique (spectrométrie de masse ou spectrométrie Raman) nécessitent un appareillage coûteux et réservé à des opérateurs qualifiés. Les méthodes colorimétriques sont plus simples, mais également plus lentes. En ce qui concerne l'amplification d'acides nucléiques, elle suppose un enchaînement de nombreuses étapes en respectant des conditions opératoires précises.

Le brevet US74665560 décrit une méthode permettant de caractériser un microorganisme basée sur l'exploitation de la diffusion et de la diffraction, par le microorganisme, d'un faisceau laser incident. Le microorganisme est disposé entre une source de lumière laser et un capteur d'image. Sous l'effet d'une illumination par le faisceau laser, on acquiert une image sur laquelle des figures de diffractions apparaissent, ces dernières constituant une signature du microorganisme observé. Le brevet US8787633 décrit une méthode répondant au même objectif. Ces documents décrivent une méthode d'identification bactérienne qui semble prometteuse, mais elle devient inapplicable dès lors que le milieu dans lequel sont disposées les bactéries est opaque, coloré ou diffusant. En effet, ces méthodes utilisent une image formée selon une configuration dite en transmission, dans laquelle l'échantillon est disposé entre une source de lumière et un capteur d'image. L'obtention d'une image exploitable est soumise à l'utilisation d'un échantillon suffisamment transparent. Ainsi, cette méthode n'est pas compatible avec des échantillons comportant un milieu de culture coloré, par exemple le milieu connu sous l'acronyme COS (Columbia Blood Ship) comportant une gélose columbia au sang de mouton. Elle n'est également pas applicable à un milieu diffusant de type CLED (Cystine Lactose Electrolyte Deficient), ou un milieu opaque de type gélose chocolat. Or, de tels milieux de culture sont fréquemment utilisés dans le diagnostic clinique.

La demande de brevet WO2016/097063 adresse en partie ce problème, en proposant un procédé d'observation de microorganismes dans lequel on forme non pas une image selon une configuration en transmission, mais selon une configuration en rétrodiffusion. L'échantillon est illuminé par un faisceau laser. Le rayonnement rétrodiffusé est focalisé, par une optique de collection, sur un capteur d'image. Le document WO2016/054408 décrit une configuration similaire, le rayonnement rétrodiffusé étant collecté par un capteur d'image CMOS dont la surface active peut atteindre 17,28 cm².

La publication Kim Huisung et al "Reflected scatterometry for non invasive interrogation of bacterial colonies", International society for optical engineering, SPIE, vol.21, n° 10, oct 2016, décrit également un dispositif fonctionnant selon une configuration en rétrodiffusion. Selon cette configuration, un écran plan est disposé entre l'échantillon est un capteur d'image. L'écran permet une rétroprojection du rayonnement rétrodiffusé par l'échantillon. Ce dernier est illuminé par un faisceau laser, réfléchi par une lame réfléchissante avant d'atteindre l'échantillon. La surface de la lame réfléchissante est de 25 cm². L'utilisation d'un écran translucide est également décrite dans US5241369.

Les inventeurs ont mis en œuvre ce procédé décrit dans WO2016/097063 et ont constaté certaines limitations, décrites ci-après.

L'objectif de l'invention est de surmonter ces limitations, en proposant un procédé d'observation et de caractérisation de microorganismes selon une configuration en rétrodiffusion. L'invention est particulièrement adaptée à un échantillon opaque, tout en restant naturellement applicable à des échantillons transparents. Elle permet une observation et une caractérisation de colonies de microorganismes à différents stades de développement, qu'il s'agisse de microcolonies ou de macrocolonies. Un autre avantage est qu'elle est simple à mettre en oeuvre et robuste, et ne requiert pas une instrumentation coûteuse. Par ailleurs, le procédé mis en œuvre est non destructif. Il peut être appliqué sur une colonie, dans son milieu de culture, sans nécessiter de prélèvement. Enfin, l'analyse est rapide, de l'ordre de la seconde.

### EXPOSE DE L'INVENTION

L'invention concerne tout d'abord un dispositif pour observer un objet, présent dans un échantillon, selon la revendication 1, le dispositif comportant:
- un support, apte à recevoir l'échantillon ;
- une source de lumière, apte à émettre un faisceau lumineux, dit faisceau lumineux indicent, pour illuminer l'objet ;
- un capteur d'image, pour acquérir une image représentative d'un rayonnement rétrodiffusé par l'objet sous l'effet d'une illumination par le faisceau lumineux incident;
- un écran, s'étendant en regard du support, de manière à être exposé à un rayonnement rétrodiffusé par l'objet lorsque ce dernier est illuminé par le faisceau lumineux incident, de façon à former, sur l'écran, une image, dite diffractogramme, représentative du rayonnement rétrodiffusé ;
- l'écran comportant une première face, exposée au rayonnement rétrodiffusé ;
- le capteur d'image étant configuré pour acquérir une image du diffractogramme formé sur l'écran.

La source de lumière peut notamment être une source de lumière laser. Le dispositif peut comporter une optique de collimation, de manière à ce que le faisceau lumineux émis par la source de lumière soit collimaté. Le dispositif peut comporter une optique d'expansion de faisceau, de manière à ajuster le diamètre du faisceau laser à la taille et à la morphologie de l'objet analysé.

L'objet peut être une colonie de microorganismes, par exemple une colonie bactérienne, auquel cas l'écran permet l'obtention d'un diffractogramme dont la taille est suffisamment importante pour caractériser une colonie à un stade de développement suffisamment avancé.

Selon l'invention, l'aire de la première face de l'écran est supérieure à 100 cm².

Le dispositif comporte un élément réfléchissant, disposé entre l'écran et l'objet, apte à réfléchir une partie du faisceau lumineux incident selon un axe d'incidence perpendiculaire ou sensiblement perpendiculaire au plan de l'échantillon, l'élément réfléchissant étant solidaire de la première face de l'écran. Cela permet d'éviter une perturbation du diffractogramme formé sur l'écran par un bras, portant l'élément réfléchissant, s'étendant transversalement au rayonnement de rétrodiffusion.

Le dispositif peut comporter l'une quelconque des caractéristiques décrites ci-dessous, prises isolément ou selon les combinaisons techniquement réalisables :
- L'écran comporte une deuxième face, de façon que le diffractogramme formé sur la première face apparaisse sur la deuxième face ; l'écran s'étend alors entre le capteur d'image et le support, de telle sorte que le capteur d'image est couplé à la deuxième face par une optique de focalisation. L'écran agit alors en tant que rétro-écran, en transmettant le diffractogramme, projeté sur la première face, vers la deuxième face.
- La distance entre l'élément réfléchissant et l'écran est inférieure à 2 cm.
- La surface de l'élément réfléchissant est inférieure à 4 cm² ou à 2 cm² ou à 1 cm².
- L'écran est translucide.
- L'écran comporte un guide de lumière, par exemple une fibre optique, pour transporter une lumière entre la première face et la deuxième face. L'écran peut comporter plusieurs fibres optiques s'étendant entre la première face et la deuxième face.
- L'écran est une partie photosensible du capteur d'image, la partie photosensible permettant une conversion du rayonnement rétrodiffusé en porteurs de charges.
- L'écran transmet moins de 90% du rayonnement rétrodiffusé de la première face vers la deuxième face.
- L'écran est mobile par rapport au support, la distance entre le support et l'écran pouvant être ajustée.
- Le faisceau lumineux incident se propage entre l'élément réfléchissant et l'objet autour d'un axe dit axe d'incidence, le dispositif comportant un réflecteur dit annulaire, s'étendant autour de l'axe d'incidence, entre l'échantillon et l'écran, le réflecteur annulaire étant apte à réfléchir une partie du rayonnement rétrodiffusé vers l'écran.
- L'écran est incurvé, notamment vers l'échantillon (ou l'objet).

L'invention concerne aussi un procédé d'observation d'un objet présent dans un échantillon,selon la revendication 12, l'échantillon s'étendant face à un écran comportant une première face, le procédé comportant les étapes suivantes :
a) illumination de l'objet à l'aide d'un faisceau lumineux incident émis par une source de lumière, le faisceau lumineux incident se propageant jusqu'à l'objet ;
b) exposition d'une première face d'un écran à un rayonnement lumineux rétrodiffusé par l'échantillon, sous l'effet de l'illumination, de façon à former, sur ladite première face, une image, dite diffractogramme, représentative dudit rayonnement rétrodiffusé ;
c) acquisition d'une image du diffractogramme, formé sur l'écran, par un capteur d'image.

Selon l'invention, le dispositif comporte un élément réfléchissant, disposé entre l'écran et l'objet, l'élément réfléchissant dirigeant tout ou partie du faisceau lumineux incident, émis par la source, vers l'objet. L'élément réfléchissant est relié à la première face de l'écran.

Le procédé peut comporter l'une quelconque des caractéristiques décrites ci-dessous, prises isolément ou selon les combinaisons techniquement réalisables :
- La surface de l'élément réfléchissant est inférieure à 5 cm², ou à 2 cm², ou à 1 cm².
- L'écran est incurvé, en s'incurvant notamment vers l'échantillon.
- L'écran est translucide.
- L'écran comporte au moins un guide de lumière, notamment une fibre optique, s'étendant entre la première face et la deuxième face.
- Une face de l'écran est structurée de façon à former une lentille.
- L'écran transmet moins de 95% ou 90% du rayonnement rétrodiffusé.
- L'écran comporte une deuxième face, l'écran s'étendant entre le capteur d'image et l'échantillon, de telle sorte que le capteur d'image est optiquement couplé à la deuxième face par une optique de focalisation, l'écran étant tel que le diffractogramme formé sur la première face apparaisse sur la deuxième face.
- Le procédé comporte, suite à l'étape c), une étape d'ajustement de la distance entre l'échantillon et l'écran en fonction de l'image acquise par le capteur d'image, les étapes a) à c) étant répétées après l'ajustement de ladite distance.
- Le procédé comporte une étape d) de caractérisation de l'objet à partir de l'image acquise par le capteur d'image, ou à partir de l'image résultante. La caractérisation de l'image peut comporter :
   une détermination de caractéristiques de l'image ;
   une identification de l'objet à l'aide desdites caractéristiques et de caractéristiques de calibration établies en mettant en œuvre les étapes a) à c) du procédé à l'aide d'un échantillon étalon.
- L'objet comporte un microorganisme. L'objet peut notamment regrouper plusieurs microorganismes formant une colonie. L'objet peut être une colonie bactérienne.
- Le procédé est mis en œuvre avec un dispositif tel que décrit dans la demande.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

### FIGURES

La figure 1A représente un dispositif d'observation de microorganismes selon l'art antérieur.
Les figures 1B et 1C illustrent des distributions spatiales de rayonnements de rétrodiffusion émanant respectivement de deux objets différents.
Les figures 2A et 2B représentent un premier mode de réalisation de l'invention. La figure 2C montre un exemple d'écran pouvant être mis en œuvre dans le premier, le deuxième ou le troisième mode de réalisation. La figure 2D est un détail de la figure 2A. Les figures 2E et 2F illustrent une variation de la distance entre l'écran et l'échantillon. La figure 2G est un exemple de système optique de mise en forme du faisceau laser.
La figure 3A illustre un deuxième mode de réalisation. La figure 3B montre un exemple, qui ne fait pas partie de la présente invention.
Les figures 3C et 3D représentent des variantes applicables à tous les modes de réalisation. La figure 3E représente une variante selon laquelle l'écran est incurvé.
Les figures 4A et 4B montrent un diffractogramme d'une colonie bactérienne, selon deux agencements différents du dispositif.
Les figures 5A, 5B et 5C illustrent un procédé pour déplacer une colonie bactérienne observée de façon à la centrer par rapport au faisceau lumineux incident et à l'écran. La figure 5A est un diffractogramme légèrement décentré. La figure 5B résulte de l'application d'un filtre au diffractogramme représenté sur la figure 5A. La figure 5C représente le diffractogramme de la figure 5A après recentrage.
La figure 6 illustre un mode de réalisation, dit de haute dynamique, selon lequel différentes images sont combinées.
Les figures 7A, 7B, 7C, 7D, 7E et 7F représentent des diffractogrammes de différents types de colonies bactériennes.
Les figures 8A et 8B montrent une image au microscope et un diffractogramme d'une colonie bactérienne. Les figures 8C et 8D montrent une image au microscope et un diffractogramme d'une autre colonie bactérienne.
Les figures 9A, 9B et 9C sont des diffractogrammes de colonies bactériennes formées sur différents milieux de culture.
La figure 9D représente le diffractogramme d'une colonie bactérienne formée sur un tapis de bactéries différentes de celles formant la colonie bactérienne observée.
La figure 10 montre une cinétique d'évolution temporelle d'un diffractogramme correspondant à une même colonie bactérienne.
La figure 11A représente un dispositif expérimental combinant un écran plat et un écran incurvé.
La figure 11B est une image d'une colonie bactérienne observée à l'aide du dispositif schématisé sur la figure 11A.

### EXPOSE DE MODES DE REALISATION PARTICULIERS

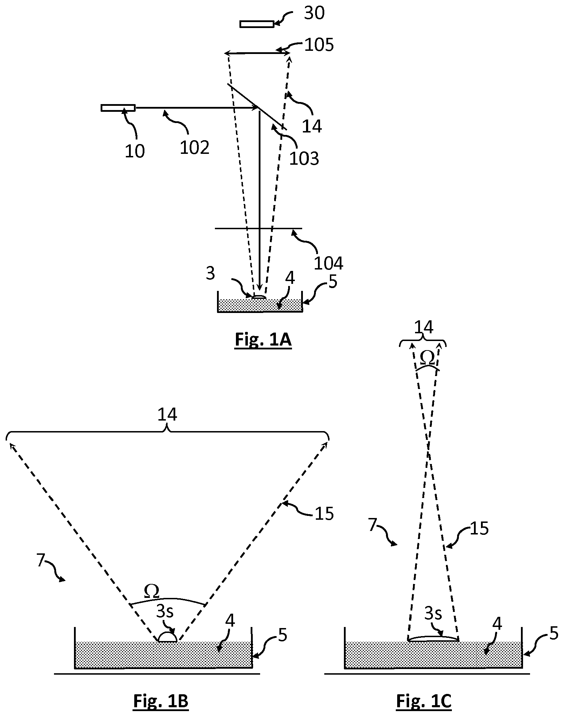

On a représenté, sur la figure 1A, un dispositif d'observation de microorganismes tel que décrit dans la demande de brevet WO2016/097063. Une source de lumière laser 10 émet un faisceau lumineux polarisé rectilignement 102 se propageant jusqu'à un objet 3 à caractériser, par exemple une colonie bactérienne disposée à la surface d'un milieu de culture 4. Avant d'atteindre la colonie bactérienne 3, le faisceau lumineux polarisé 102 est dévié par une lame semi-réfléchissante 103, de façon à se propager selon une direction, dite direction d'incidence, sensiblement perpendiculaire à la surface du milieu de culture 4. Le faisceau lumineux 102 traverse une lame quart d'onde 104 avant d'atteindre la colonie bactérienne 3. Le faisceau lumineux 102 interagit avec la colonie bactérienne 3, ce qui résulte en la formation d'un rayonnement rétrodiffusé 14 se propageant selon une direction sensiblement opposée à la direction d'incidence. Le rayonnement rétrodiffusé 14 est formé par de multiples interactions du faisceau lumineux 102 avec la colonie 3, combinant les effets de diffraction et de diffusion élastique dans la colonie. Le rayonnement rétrodiffusé 14 traverse la lame quart d'onde 104, puis la lame semi-réfléchissante 103, avant d'être focalisé par un système optique 107 vers un capteur d'image 30. L'image formée sur le capteur d'image, désignée par le terme diffractogramme, est représentative du rayonnement de rétrodiffusion 14. Le diffractogramme peut constituer une signature de la colonie bactérienne, permettant une identification des bactéries formant la colonie. Ce dispositif, représentant l'art antérieur, a été mis en œuvre par les inventeurs. Ces derniers ont montré que ce dispositif ne permettait pas une observation satisfaisante de certaines colonies bactériennes.

En effet, le rayonnement rétrodiffusé 14 est émis selon une plage angulaire variable en fonction du type de microorganisme observé. Certaines colonies bactériennes, par exemple une colonie de staphylocoques, se développent en formant progressivement une surface externe 3s ayant une forme proche d'un hémisphère délimité par un milieu ambiant 7, par exemple de l'air. Un tel cas de figure est illustré sur la figure 1B. Dans ce type de configuration, le rayonnement rétrodiffusé 14 se propage de façon divergente, en formant un cône 15 selon une plage angulaire Ω élevée. Par élevée, on entend comprenant des angles supérieurs à 65° voire à 85°. Cela est notamment dû à la réfraction du rayonnement rétrodiffusé lorsqu'il franchit la surface 3s pour être réfracté dans le milieu ambiant 7. A l'inverse, comme représenté sur la figure 1C, d'autres colonies bactériennes se développent en formant progressivement une surface 3s plane, dont le rayon de courbure est élevé. De ce fait, le rayonnement rétrodiffusé 14 est réfracté dans le milieu ambiant 7 et se propage dans ce dernier selon un faisceau convergent, formant un cône 15, d'angle au sommet Ω. Les bactéries de type *enterobacteriace* forment des colonies présentant une telle morphologie. Ainsi, en fonction du type des microorganismes observés et de leur stade de développement, la morphologie d'une colonie évolue, conditionnant la distribution spatiale du rayonnement rétrodiffusé 14. Une limite du dispositif décrit dans WO2016/097063 est que le champ d'observation est faible et fixe, ne convenant pas aux colonies bactériennes dont la forme se rapproche de l'exemple de la figure 1B. Les inventeurs ont conçu un dispositif d'observation tenant compte de la variabilité de la distribution spatiale du rayonnement rétrodiffusé 14. Plus précisément, le dispositif, selon l'invention, dispose d'un champ d'observation pouvant s'adapter au microorganisme observé. En effet, les inventeurs ont établi que pour les microorganismes générant un rayonnement rétrodiffusé dont la distribution spatiale est illustrée sur la figure 1B, un champ d'observation très large peut être nécessaire.

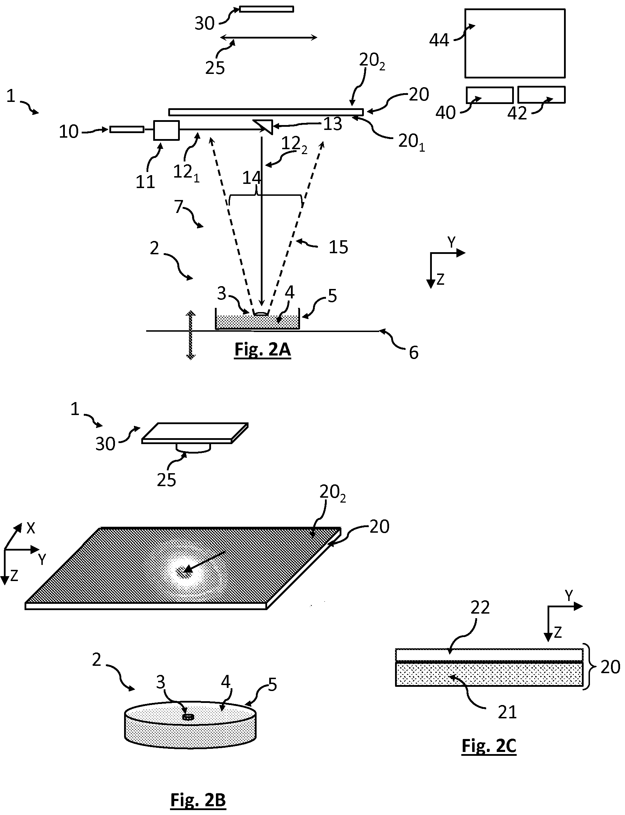

La figure 2A représente un premier mode de réalisation d'un dispositif 1 selon l'invention. Il s'agit ici d'un mode de réalisation préféré. Le dispositif comporte une source de lumière 10, apte à émettre un faisceau lumineux 12, dit faisceau incident, se propageant jusqu'à un échantillon 2 comportant un objet 3 à caractériser. La source de lumière 10 est de préférence temporellement et spatialement cohérente. La source de lumière 10 est de préférence une source laser. Selon une variante, la source de lumière peut être une diode électroluminescente ou une source de lumière blanche. Il est alors préférable que la source de lumière soit suffisamment ponctuelle pour être spatialement cohérente. Cela peut être obtenu en associant la source de lumière 10 à un filtre spatial, par exemple un diaphragme, ou à une fibre optique. La source de lumière 10 peut également être associée à un filtre passe-bande, de manière à obtenir une bande spectrale d'émission Δλ suffisamment étroite, et de préférence inférieure à 50 nm voire 10 nm.

Le faisceau incident 12 émis par la source de lumière et se propageant vers l'objet 3 est de préférence un faisceau parallèle, dont le diamètre peut avantageusement être ajusté. Le diamètre du faisceau incident 12 est de préférence compris entre 100 µm et 10 mm. L'ajustement du diamètre permet de s'adapter à la taille de l'objet 3 à caractériser. Ainsi, lorsque l'objet 3 est une colonie bactérienne, cela permet d'ajuster la taille du faisceau incident 12 à la morphologie de la colonie, cette dernière dépendant du type de bactérie et du stade de développement. Un système optique de mise en forme 11 peut être disposé entre la source de lumière 10 et l'objet 3. Le système optique de mise en forme 11 peut permettre un ajustement du diamètre du faisceau incident 12. Il peut également uniformiser une distribution spatiale de l'énergie dans le faisceau incident 12, de façon à ce que l'intensité lumineuse soit plus homogène dans le faisceau.

L'objet à caractériser 3 peut être un microorganisme ou un ensemble de microorganismes formant une colonie. Le microorganisme peut être une bactérie, une levure, un champignon ou une microalgue. L'objet à caractériser peut également être un groupe de cellules, formant par exemple un amas. L'objet à caractériser peut être en contact avec un milieu de culture 4, en étant disposé dans ce dernier ou à la surface de ce dernier. Le milieu de culture 4 est confiné dans une enceinte 5. Le milieu de culture 4 et/ou l'enceinte 5 peut être opaque ou translucide. En particulier, il n'est pas nécessaire que le milieu de culture 4 et l'enceinte 5 soient transparents, condition imposée par les procédés basés sur des configurations en transmission décrites en lien avec l'art antérieur. L'ensemble formé par l'enceinte 5, le milieu de culture 4 et l'objet 3 constitue l'échantillon 2, ce dernier reposant sur un support 6. Dans l'exemple représenté, le support est une platine plane mobile en translation selon un axe Z, dit axe d'incidence. L'invention est particulièrement adaptée aux échantillons comportant un milieu de culture 4 opaque. Lorsque le milieu de culture 4 n'est pas suffisamment opaque, il est préférable que l'enceinte 5 soit opaque, et de préférence absorbante, de façon à minimiser des réflexions parasites. L'enceinte 5 peut comporter un couvercle, sous réserve que ce dernier soit transparent. Lorsque l'enceinte 5 est transparente, il est préférable qu'elle soit disposée sur un support 6 opaque ou translucide. Un tel support évite les réflexions parasites.

Le dispositif comporte un élément réfléchissant 13, par exemple un miroir, apte à diriger le faisceau lumineux incident 12, émis par la source, selon un axe d'incidence Z sensiblement perpendiculaire à la surface 3s de l'objet 3 à observer, ou sensiblement perpendiculaire à un plan XY, dit plan de l'échantillon, selon lequel s'étend le milieu de culture 4 de l'échantillon 2. Par sensiblement perpendiculaire, on entend perpendiculaire à une tolérance angulaire près, cette dernière étant de préférence inférieure à ±30°, ou de préférence inférieure à ±20°. Ainsi, le faisceau lumineux incident 12 atteint l'objet 3 selon un angle d'incidence sensiblement égal à 90°, à la tolérance angulaire près. Dans l'exemple représenté, le faisceau lumineux incident comporte une première composante 12₁, entre la source de lumière 10 et l'élément réfléchissant 13, et une deuxième composante 12₂, entre l'élément réfléchissant 13 et l'objet 3. Le faisceau incident 12 atteignant l'objet est de préférence centré par rapport à l'objet 3, selon le plan de l'échantillon XY.

Sous l'effet de l'illumination par le faisceau incident 12, l'objet 3 émet un rayonnement rétrodiffusé 14, se propageant selon ou autour d'un axe central de rétropropagation -Z parallèle à l'axe d'incidence Z, et de sens opposé à ce dernier. D'une façon générale, le terme rayonnement rétrodiffusé désigne un rayonnement se propageant selon un axe de propagation comportant une composante opposée à l'axe d'incidence Z. Le rayonnement rétrodiffusé 14 résulte de l'interaction des photons du faisceau incident 12 avec l'objet 3, ce dernier présentant un indice de réfraction plus élevé que l'indice de réfraction du milieu ambiant 7 dans lequel se propage le faisceau incident, le milieu ambiant 7 étant généralement de l'air. Du fait de l'angle d'incidence, la majeure partie du faisceau incident 12 pénètre dans l'objet 3 en formant un faisceau incident réfracté. Le faisceau incident 12 réfracté dans l'objet 3 subit une ou plusieurs diffusions élastiques dans l'objet, et peut générer des ondes de diffraction. Un rayonnement rétrodiffusé 14 émane de l'objet en se propageant à travers la surface 3s, comme décrit en lien avec les figures 1B et 1C. Le rayonnement rétrodiffusé est réfracté dans le milieu ambiant 7, puis se propage, autour de l'axe de rétropropagation -Z, jusqu'à un écran 20, sur lequel il forme une image I₂₀, dite diffractogramme, représentative du rayonnement de rétrodiffusion. L'axe de rétropropagation -Z est coaxial de l'axe d'incidence Z selon lequel le faisceau incident atteint l'objet. Dans la littérature anglosaxonne, le diffractogramme est usuellement désigné par le terme "scattergram", ou "scattering pattern", que l'on pourrait également traduire par le terme diffusiogramme. On note qu'aucune optique de focalisation ou de formation d'image n'est disposée entre l'objet 3 et l'écran 20.

La surface de l'élément réfléchissant 13 est la plus faible possible, de façon à ne pas interférer avec le rayonnement rétrodiffusé 14 émanant de l'objet 3. Elle est de préférence inférieure à 5 cm², et encore de préférence inférieure à 2 cm², voire inférieure à 1 cm². La surface de l'élément réfléchissant 13 est de préférence adaptée au diamètre du faisceau émis par la source de lumière 10.

L'écran 20 est apte à collecter le rayonnement 14 rétrodiffusé par l'objet 3 lorsqu'il est illuminé par le faisceau lumineux 12. Le terme écran désigne un élément dont une première face 20₁ collecte le rayonnement rétrodiffusé 14, ce dernier étant projeté sur ladite première face 20₁. Ainsi, le diffractogramme I₂₀ se forme sur la première face 20₁ de l'écran 20. L'écran 20 s'entend selon le plan de l'échantillon XY, selon une aire d'au moins 100 cm², mais il est préférable que l'aire soit supérieure à 200 cm², et par exemple de 400 cm², soit un carré de 20 cm de côté.

Le dispositif comporte un capteur d'image 30, pour acquérir une image I₃₀ du diffractogramme I₂₀ formé sur l'écran. Le capteur d'image 30 peut notamment être un capteur matriciel comportant des pixels arrangés selon une matrice, chaque pixel formant un photodétecteur élémentaire. Le capteur d'image 30 est par exemple un capteur de type CCD ou CMOS. Le capteur d'image 30 est relié à un processeur 40, par exemple un microprocesseur, comportant une mémoire 42 dans laquelle sont stockées des instructions de traitement d'image, permettant l'analyse de l'image acquise par le capteur d'image 30 en vue de caractériser l'objet 3. Le processeur 40 peut également permettre un déplacement du support 6 par rapport à l'écran 20, comme expliqué ci-après. Un moniteur 44 permet de visualiser l'image acquise.

Dans le mode de réalisation illustré sur la figure 2A, afin de permettre une projection du diffractogramme sur l'écran 20, l'écran 20 n'est pas totalement transparent : il interagit avec le rayonnement diffusé 14, par absorption et/ou diffusion. De préférence, l'écran transmet jusqu'à 80%, voire 90% ou 95% du rayonnement rétrodiffusé, la partie non transmise étant absorbée ou diffusée. Les inventeurs estiment qu'une transmittance de l'ordre de 75% est optimale. Par transmittance, on entend un ratio entre une intensité d'un rayonnement transmis par l'écran et une intensité d'un rayonnement incident à l'écran. La transmittance de l'écran est de préférence inférieure à 95%, voire inférieure à 90% ou 80%. L'opacité est définie comme étant l'inverse de la transmittance. L'écran 20 comporte une deuxième face 20₂, s'étendant, de préférence, parallèlement à la première face 20₁.L'écran 20 est configuré de telle sorte que l'image projetée sur la première face 20₁, en l'occurrence le diffractogramme, apparaisse également, par transparence et/ou par diffusion, sur la deuxième face 20₂. L'écran 20 fonctionne alors selon un rétroécran, ou écran de rétroprojection, en étant interposé entre la source du rayonnement diffusé, en l'occurrence l'objet 3, et le capteur d'image 30. L'écran 20 peut être translucide, le terme translucide désignant un matériau non transparent, c'est-à-dire à travers lequel on ne peut pas distinguer nettement des éléments, mais laissant passer la lumière. Il s'agit par exemple d'un substrat de papier calque, d'un substrat comportant des éléments diffusants, par exemple des microbilles, ou encore d'une toile ou d'une plaque de verre dépoli. Lorsque l'écran comporte des microbilles, il peut s'agir de microbilles en polycarbonate. Des écrans de rétroprojection, sous la forme de toiles, convenant à cette application sont par exemple fournis par la société Multivision, sous les références "retro gris" ou "retro crème". Lorsque l'écran 20 est un papier calque, il peut comporter une surface rugueuse dont la rugosité Bendtsen atteint 100 à 300 ml/mm, la rugosité Bendtsen étant déterminée selon la norme NF 8791-2. Le diffractogramme formé sur la première face 20₁ apparaît sur la deuxième face 20₂, comme représenté sur la figure 2B.

Le dispositif comporte une optique de focalisation 25, permettant de focaliser le diffractogramme I₂₀ formé sur la deuxième face 20₂ de l'écran 20, de telle sorte que l'image I₃₀ acquise par le capteur d'image corresponde à ce diffractogramme. De préférence, le capteur d'image 30 s'étend parallèlement à l'écran 20, et l'optique de focalisation 25 comporte un axe optique coaxial de l'axe de rétropropagation -Z (ou de l'axe d'incidence Z). L'image formée par le capteur d'image correspond donc au diffractogramme I₂₀ formé sur l'écran, sans déformation.

Selon une variante, l'écran 20 comporte un composant optique structuré, définissant par exemple une lentille de Fresnel. Une lentille de Fresnel comporte des structures annulaires concentriques agencées pour focaliser une image de grand diamètre selon une distance focale courte. La société DNP commercialise des écrans destinés à des applications en rétrodiffusion, basés sur des lentilles optiques structurées dans une face ou dans les deux faces d'un écran. Ces écrans sont désignés par le terme "optical rear projection screens". De tels écrans permettent d'augmenter la quantité de signal collectée par le capteur d'image.

Selon une variante, l'écran 20 comporte plusieurs guides de lumières s'étendant entre la première face 20₁ et la deuxième face 20₂, pour transporter le diffractogramme de la première face 20₁ vers la deuxième face 20₂. Il peut s'agir d'un panneau à fibres optiques, comportant un réseau de fibres optiques s'étendant les unes à côté des autres, entre la première face 20₁ et la deuxième face 20₂. La dimension, selon le plan XY, d'un tel écran peut atteindre plusieurs centaines de cm², par exemple 32,5 cm x 32,5 cm. Le diamètre de chaque fibre optique est compris entre 5 µm et 25 µm, l'ouverture numérique étant comprise entre 0.92 et 1. De tels panneaux sont par exemple commercialisés par la société Schott.

La figure 2C représente un écran formé de deux couches : une couche inférieure 21, définissant la première face 20₁ de l'écran, et une couche supérieure 22 définissant la deuxième face de l'écran 20₂. La couche inférieure 21 peut être diffusante, en étant par exemple constituée d'une plaque dépolie en verre ou en plastique, la surface dépolie correspondant à la première face 20₁.La couche supérieure 22 peut former une lentille de Fresnel ou une plaque transparente en verre, faisant office de couche de protection.

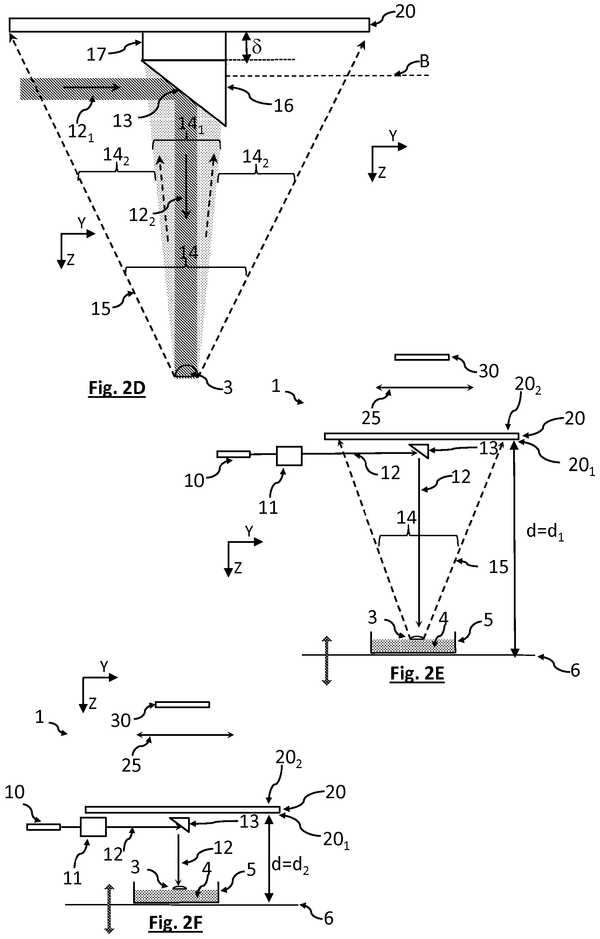

La figure 2D représente un détail de l'élément réfléchissant 13, ainsi que le rayonnement incident 12 et le rayonnement rétrodiffusé 14. Le rayonnement incident 12 comporte une première composante 12₁, se propageant entre la source de lumière 10 et l'élément réfléchissant 13. Il comporte une deuxième composante 12₂ se propageant de l'élément réfléchissant 13 jusqu'à l'objet 3. On a également représenté le rayonnement rétrodiffusé 14 émanant de l'objet 3, et prenant la forme d'un cône 15 d'angle au sommet Ω. Il comporte une première composante, notée 14₁, dite composante de réflexion, correspondant essentiellement à une réflexion spéculaire du faisceau incident 12 à la surface de l'échantillon, à laquelle s'ajoute l'ordre 0 de la diffraction. Il comporte une deuxième composante 14₂, s'étendant autour de la première composante 14₁, la deuxième composante comportant l'information exploitable pour caractériser l'objet 3. L'élément réfléchissant 13 est dimensionné en fonction du diamètre du faisceau 12₁ émis par la source de lumière. Il s'agit par exemple d'un prisme de côté 10 à 15 mm, incliné à 45° par rapport à l'orientation du faisceau 12₁ provenant de la source de lumière 10. L'élément réfléchissant 13 est solidaire de l'écran 20. Cela permet d'éviter qu'un bras B, destiné à maintenir l'élément réfléchissant, ne s'étende dans le cône 15 de propagation du rayonnement de rétrodiffusion 14, ce qui entraînerait une dégradation du diffractogramme formé sur l'écran 20. La distance δ entre l'élément réfléchissant 13 et l'écran 20 est de préférence supérieure à 1mm. Une distance δ trop faible, par exemple inférieure à 1 mm peut entraîner une interaction du faisceau laser 12₁ émis par la source et se propageant jusqu'à l'élément réfléchissant 13, avec l'écran 20. Il est préférable que la distance δ soit inférieure à 10 mm ou 20 mm, voire 30 mm, de façon à ne pas gêner une translation de l'échantillon 2 en direction de l'écran 20, comme décrit ci-après. L'élément réfléchissant 13 est de préférence traité anti-reflet. L'élément réfléchissant 13 peut comporter une surface arrière opaque 16, de façon à bloquer une propagation d'un rayonnement non réfléchi. Cela permet d'éviter des fuites de lumière. L'élément réfléchissant est relié à l'écran 20 par un support de liaison 17. Le support de liaison 17 s'étend, entre l'élément réfléchissant et l'écran, parallèlement à l'axe Z du faisceau incident 12 atteignant l'échantillon, en étant avantageusement coaxial du faisceau incident 12 atteignant l'échantillon.

De préférence, contrairement au dispositif décrit dans la demande WO2016/097063, le rayonnement rétrodiffusé 14 se propageant vers l'écran 20 est bloqué soit par l'élément réfléchissant 13, soit par le support de liaison 17. Cela bloque une transmission de la première composante 14₁ (composante de réflexion) du rayonnement rétrodiffusé vers l'écran. Or, comme précédemment indiqué, la composante de réflexion 14₁ représente essentiellement une réflexion spéculaire du faisceau incident 12 sur l'objet 3 ; elle ne comporte pas, ou peu, d'information utile pour caractériser l'objet 3 observé. De plus, cette première composante est généralement intense. Sa non-transmission vers l'écran 20, permet de supprimer une contribution intense et peu informative sur le diffractogramme. Cela améliore la dynamique du diffractogramme. Le masquage de la composante de réflexion 14₁ apparaît, sur les diffractogrammes, sous la forme d'un disque sombre, ce dernier étant une ombre de l'élément réfléchissant 13, ou du support de liaison 14. Cette ombre est désignée par une flèche noire sur le diffractogramme représenté sur la figure 2B. L'élément l'élément réfléchissant 13 et/ou le support de liaison 17 absorbent au moins 50%, et avantageusement au moins 80%, voire 90% du rayonnement rétrodiffusé 14 émis par l'objet. Leur taille est ajustée de manière à ce qu'ils ne masquent que la composante de réflexion 14₁, et non la composante 14₂ du rayonnement rétrodiffusé, comportant l'information utile.

La distance d entre l'échantillon 2 et l'écran 20 est avantageusement variable, comme illustré sur les figures 2E et 2F. En effet, comme précédemment indiqué, la distribution spatiale du rayonnement de rétrodiffusion 14 peut varier, ce dernier pouvant prendre la forme d'un cône 15 plus ou moins ouvert, et s'étendant, à partir de l'objet de façon divergente ou convergente. De ce fait, le support 6 de l'échantillon peut être monté sur une platine de translation autorisant une translation parallèlement à l'axe d'incidence Z. Les figures 2E et 2F représentent un échantillon 2 situé respectivement à une première distance d = d₁ et à une deuxième distance d = d₂ de l'écran 20, avec d₁ > d₂. Le déplacement du support 6 peut être commandé par le processeur 40. La plage de variation de la distance est typiquement de 3 cm à 20 cm, voire 30 cm. La distance est déterminée en fonction du diffractogramme formé sur l'écran 20, de façon que le diffractogramme s'étende selon une surface la plus grande possible, tout en restant compatible avec le champ d'observation du capteur d'image, ce dernier dépendant de la dimension du capteur d'image 30 et du système optique de focalisation 25.

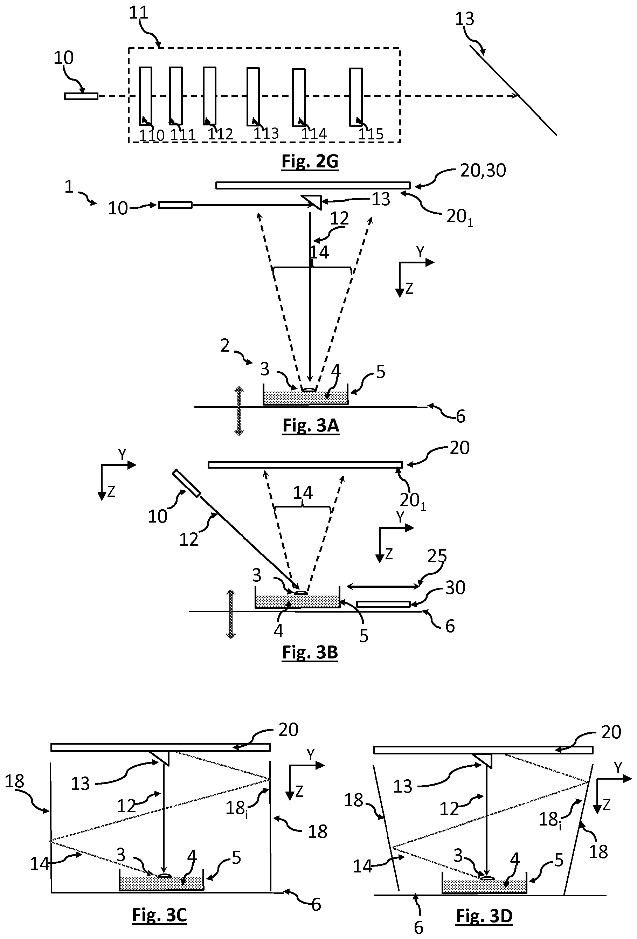

Le réglage de la distance peut se faire manuellement, ou en mettant en œuvre un algorithme basé sur une reconnaissance du contour délimitant le diffractogramme. Un tel algorithme peut par exemple utiliser un filtrage de Canny. Lorsque ce contour a été détecté, la distance est ajustée de façon que l'aire du diffractogramme, sur l'écran 20, dépasse une valeur seuil prédéterminée. L'ajustement de la distance d permet de prendre en compte la variabilité du rayonnement de rétrodiffusion en fonction des différents types d'objets à caractériser. Selon un mode de réalisation, lorsqu'une distance optimale a été déterminée, permettant de maximiser l'aire du diffractogramme projeté sur l'écran, une image du diffractogramme est acquise. La distance est ensuite augmentée, de façon à vérifier l'absence de rayonnement rétrodiffusé à l'extérieur du diffractogramme correspondant à la distance optimale, préalablement observé. De préférence, le support 6 est également mobile dans le plan de l'échantillon XY. Cela permet d'effectuer un centrage du faisceau lumineux incident 12 sur l'objet 3. Cela permet d'effectuer une analyse quelle que soit la position de l'objet 3 dans l'échantillon 2. Un tel centrage peut être ajusté sur un critère de symétrie du diffractogramme. En effet, lorsque le faisceau incident est centré sur l'objet, le diffractogramme présent sur l'écran présente une symétrie de révolution. La symétrie peut par exemple être quantifiée par la forme du contour du diffractogramme Selon un deuxième mode de réalisation, représenté sur la figure 3A, l'écran 20 est formé par un capteur d'image de grande dimension, dont la surface sensible est supérieure à 100 cm², voire davantage. Selon ce mode de réalisation, l'écran 20 fait également office de capteur d'image 30. Le capteur d'image peut être un capteur tel qu'utilisé dans les dispositifs d'imagerie médicale, de type imagerie X, en étant alors couplé avec un matériau scintillateur assurant une conversion entre le rayonnement X et un rayonnement visible détectable par le capteur d'image. Ce type de capteur est sensible au rayonnement visible, tout en ayant une surface pouvant être élevée. L'écran 20 correspond à une partie photosensible du capteur d'image 30 dans laquelle les photons visibles incidents sont convertis en porteurs de charges.

Un exemple de fabrication d'un tel capteur en silicium, dont la surface de détection est supérieure à 100 cm², voire 200 cm², est donné dans le document WO2014/006214. La surface des pixels peut être comprise entre 50 µm et 200 µm. Une plaque transparente de protection de fine épaisseur, typiquement quelques millimètres, peut être disposée contre l'écran 20. Un tel mode de réalisation peut permettre un gain significatif en sensibilité par rapport au premier mode de réalisation, le coût étant cependant plus élevé.

Selon un exemple, qui ne fait pas partie de la présente invention, représenté sur la figure 3B, l'écran 20 n'est pas mis en œuvre en rétroprojection, mais en projection simple, l'échantillon 2 et le capteur d'image 30 étant disposés en regard d'une même face de l'écran 20. Selon cet exemple, le rayonnement de rétrodiffusion 14 forme un diffractogramme sur la première face de l'écran 20₁. Le capteur d'image 30 est optiquement couplé à la première face 20₁, à l'aide d'un système optique 25. Le capteur d'image 30 acquiert une image du diffractogramme projeté sur la première face. Cependant, selon cet exemple, le capteur d'image est décentré par rapport à l'écran. Cet exemple prive la possibilité de supprimer la composante de réflexion 14₁ du diffractogramme formé sur l'écran 20. De plus, cet exemple ne permet pas de centrer à la fois l'écran 20 et le faisceau lumineux incident 12 sur l'objet 3 à caractériser.

La distribution spatiale du rayonnement rétrodiffusé 14 peut varier significativement selon les objets observés. Dans certains cas, elle s'étend selon une plage angulaire très élevée de part et d'autre de l'axe d'incidence Z. C'est notamment le cas lorsque l'objet, en l'occurrence une colonie bactérienne, présente une morphologie bombée, une telle morphologie étant par exemple observée sur des colonies bactériennes de staphylocoques. Dans un tel cas de figure, la taille de l'écran 20 doit être importante pour obtenir un diffractogramme complet, et en particulier prenant en compte les grands angles de rétrodiffusion, typiquement supérieurs à 65°. Le terme angle de rétrodiffusion désigne l'angle entre un rayonnement rétrodiffusé 14 émanant de l'objet et l'axe d'incidence Z. Il est également possible d'ajuster la distance entre l'écran 20 et l'objet 3, comme précédemment indiqué. Cela permet notamment d'obtenir un diffractogramme dont le diamètre correspond à un gabarit prédéterminé, par exemple un diamètre compris entre 15 et 20 cm. La figure 3C décrit une variante permettant de conserver une taille d'écran 20 raisonnable tout en permettant une prise en compte du rayonnement de rétrodiffusion 14 émanant de l'objet sous des grands angles de rétrodiffusion. Selon cette variante, un réflecteur annulaire 18, s'étendant parallèlement à l'axe d'incidence Z, est disposé entre l'objet 3 et l'écran 20, autour de tout ou partie de l'objet 3. Le réflecteur annulaire 18 permet de réfléchir une partie du rayonnement rétrodiffusé 14 vers l'écran 20. Il peut s'agir d'un réflecteur tubulaire coaxial de l'axe d'incidence Z. La figure 3C représente un réflecteur annulaire cylindrique. Sa hauteur et son diamètre peuvent être respectivement de 6 cm et 17.5 cm. Il peut s'agir d'un cylindre dont la paroi interne 18i est réfléchissante. Par exemple, la paroi interne 18i a fait l'objet d'un dépôt d'une fine couche métallique, par exemple de l'aluminium. Le réflecteur annulaire 18 peut également être de forme conique, comme représenté sur la figure 3D. Un tel réflecteur conique peut avoir un petit diamètre égal à 19 cm, un grand diamètre égal à 20 cm, et une hauteur de 3 cm. L'angle d'inclinaison de la paroi interne, par rapport à l'axe Z, est par exemple de 13°. L'angle de la paroi interne 18i peut être dimensionné de telle sorte que le rayonnement rétrodiffusé 14 présentant l'angle de rétrodiffusion le plus élevé ne subisse qu'une seule réflexion avant d'atteindre l'écran 20. De préférence, au moins un diamètre du réflecteur annulaire 18 est supérieur à 2 fois le diamètre de l'enceinte 5.

Un espace peut être ménagé entre le réflecteur annulaire 18 et l'écran 20, de façon à permettre une propagation du faisceau lumineux incident 12 entre la source de lumière 10 et l'élément réfléchissant 13.

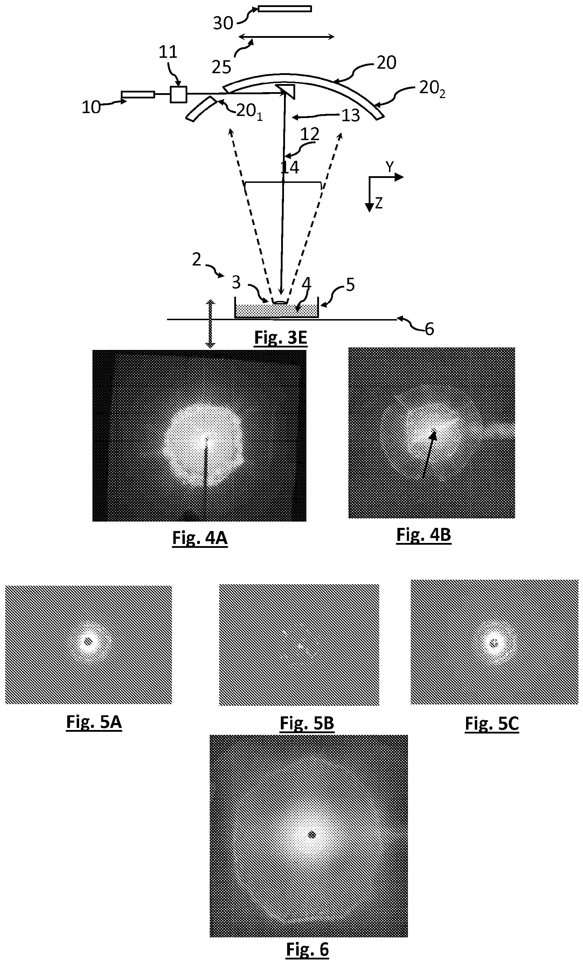

La figure 3E représente une variante selon laquelle, l'écran 20 n'est pas plan et prend une forme courbée s'incurvant vers l'échantillon 2. Cela facilite également une collecte, par l'écran, de rayonnements rétrodiffusés selon des angles de rétrodiffusion importants. La courbure de l'écran 20 peut être régulière ou non. L'écran 20 peut par exemple décrire tout ou partie d'un hémisphère. L'écran 20 peut avoir une forme de dôme. L'écran peut également décrire une courbure en présentant des facettes planes. Par s'incurvant vers l'échantillon, on entend que l'écran décrit une courbure dont le centre est compris entre l'échantillon et l'écran, ou, de façon plus générale, dont le centre est situé dans un demi-espace délimité par l'écran et comportant l'échantillon. Ainsi, l'écran a une forme concave, de façon à définir un espace s'étendant entre l'écran et l'échantillon, l'espace étant tel que quels que soient deux points dudit espace, le segment reliant lesdits points est inclus dans l'espace. Il est par exemple possible d'utiliser un écran en forme de dôme commercialisé par Draper Draper Inc, en acrylique, référence IRUS Screen, custom vaccuum formed %" acrylic, tint Cine25, coating HC. De préférence, l'élément réfléchissant est disposé à proximité de la première face 20₁ de l'écran, au niveau de l'apex de l'écran 20.

Quel que soit le mode de réalisation, un système optique de mise en forme 11 peut être associé à la source de lumière 10, de façon à former un faisceau incident 12 collimaté, selon des principes connus de l'homme du métier. La figure 2G représente un exemple de système optique de mise en forme. Il comporte une succession de composants optiques usuels : une lentille achromat 110, un sténopé 111 de 50 µm, une lentille convergente 112 et un expanseur de faisceau 113 . Le système optique de mise en forme 11 peut comprendre, de façon optionnelle, un convertisseur de faisceau de type "flat top" 114 suivi d'un réducteur de faisceau 115. L'expanseur de faisceau 113 permet d'ajuster la taille du faisceau laser, de façon à ce que cette dernière se rapproche de la taille de l'objet à observer. L'expanseur 113 peut être constitué d'un ensemble de deux lentilles à focale variable, programmable par le processeur 40. Le convertisseur de faisceau 114 permet d'ajuster la distribution de l'intensité dans le faisceau.

L'image obtenue sur le capteur d'image peut permettre de caractériser l'objet. La caractérisation peut être une identification. Pour cela, des caractéristiques de l'image sont déterminées, et comparées à des caractéristiques de calibration établies sur des objets étalons. Elles peuvent également faire l'objet d'une classification sur la base desdites caractéristiques de calibration. La demande de brevet WO2014184390 décrit un procédé de classification de colonies bactériennes basé sur une projection de l'image sur une base de polynômes de Zernike orthogonaux. D'autres algorithmes de classification, par exemple une analyse en composantes principales, sont envisageables. Une telle classification a pour objet de réduire l'information spatiale de l'image en un jeu de coordonnées, sur la base duquel l'identification du microorganisme est obtenue.

Le procédé n'étant pas destructif, plusieurs images d'une même colonie bactérienne, à différents stades d'incubation, peuvent être réalisées, de façon à apprécier la capacité de la colonie à se développer, ou à résister à un agent antibiotique ou antibactérien. Dans ce cas, la caractérisation de l'objet représente l'aptitude de ce dernier à se développer.

Le procédé peut également permettre un dénombrement d'objets présents à la surface d'un échantillon.

### Essais expérimentaux

On décrit à présent des essais expérimentaux réalisés en mettant en œuvre le premier mode de réalisation. Les principaux composants utilisés ont été les suivants :
- source de lumière 10 : source laser référence LCG FP-D-532-10C-F - fournie par Laser components.
- système optique de mise en forme 11 : lentille achromat Thorlabs AC254-030-A-ML - A280TM-A ; sténopé Thorlabs - P50S, lentille convergente Thorlabs A280TM-A.
- Enceinte de l'échantillon : boîte de Pétri de diamètre 90 mm - Biomérieux.
- Ecran translucide : Polycarbonat-diffuser L80P3-12 Luminit, ou papier calque.
- Système optique de focalisation : LM5JC10M - Kowa.
- Caméra : UI-1492ME ― IDS ou AVGT3300 ― Allied Vision.
- Element réfléchissant : miroir incliné à 45°.

L'ensemble est placé dans l'obscurité.

Au cours de ces essais, on a observé différents types de colonies bactériennes. Lors de chaque opération, le centrage du faisceau laser incident 12 sur la colonie est réalisée visuellement, par l'opérateur. Le temps de pose de chaque image acquise était compris entre 0.6 ms et 1500 ms. Certaines images ont été obtenues en sommant différentes images acquises par le capteur d'image.

Les figures 4A et 4B représentent un diffractogramme d'une colonie bactérienne *Escherichia coli* sur un milieu de culture COS (Columbia Blood Ship). L'écran utilisé est un papier calque. L'élément réfléchissant 13 est supporté soit par un bras transversal B s'étendant parallèlement à l'écran 20 (figure 4A), soit par un support 17 fixé à l'écran (figure 4B), comme représenté sur la figure 2D. Sur la figure 4A, on observe que le bras transversal B, s'étendant parallèlement à l'écran 20, coupe le rayonnement rétrodiffusé 14, ce qui produit une ombre rectiligne sombre sur le diffractogramme. On observe également que le diffractogramme comporte une zone centrale intense, saturant les pixels du capteur d'image, qui correspond à la composante de réflexion 14₁ précédemment décrite. Sur la figure 4B, on observe une tache sombre circulaire centrale, formée par l'ombre du support 17, repérée par une flèche. Cette ombre masque la composante de réflexion 14₁. Par conséquent, la dynamique de l'image est optimisée, et la zone périphérique 14₂ du diffractogramme apparaît plus nettement. Ces images justifient la disposition de l'élément réfléchissant 13 décrite en lien avec la figure 2D.

Les figures 5A à 5C illustrent un procédé de centrage de l'objet 3 par rapport au faisceau incident 12 et par rapport à l'écran. La figure 5A est une image d'un diffractogramme d'une colonie bactérienne de type *Escherichia aerogenes,* sur un milieu de culture COS, l'écran utilisé étant un papier calque. En appliquant une détection de contours de type filtre de Canny, la figure 5B a été obtenue. Le diamètre du diffractogramme est estimé à 8.2 cm. Le centre du diffractogramme a été déterminé, et l'échantillon a été déplacé de façon que le centre du diffractogramme soit placé au centre de l'image acquise par le capteur d'image (figure 5C). Cela permet un alignement de la colonie avec le faisceau incident 12 et avec l'axe optique du capteur d'image.

La figure 6 est une image, dite de haute dynamique, obtenue en acquérant 11 images d'un même diffractogramme, le temps de pose variant entre 8 ms et 495 ms. Un algorithme de type HDR, signifiant haute dynamique, a été mis en œuvre pour combiner les images acquises et former l'image de la figure 6. Cette image représente une colonie bactérienne de type *Staphylococcus epidermidis* sur un milieu de culture COS, l'écran étant un papier calque.

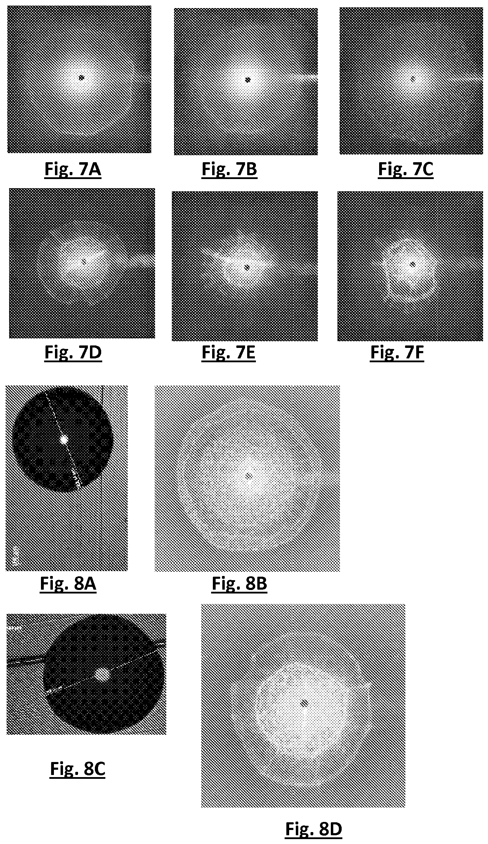

Les figures 7A à 7F sont des exemples d'images de diffractogrammes obtenus en observant des colonies bactériennes se développant sur une gélose COS préalablement décrite. Chaque diffractogramme a pour dimension 20 cm² par 20 cm². L'écran utilisé pour obtenir ces diffractogrammes était un papier calque. Les paramètres de chaque figure sont à présent listés:
- figure 7A : *Staphylococcus warneri* - diamètre du faisceau laser : 900 µm ;
- figure 7B : *Staphylococcus saprophyticus-* diamètre du faisceau laser : 900 µm ;
- figure 7C : *Staphylococcus epidermidis* - diamètre du faisceau laser : 900 µm ;
- figure 7D : *Escherichia coli* - diamètre du faisceau laser : 1800 µm ;
- figure 7E : *Pseudomonas putida* - diamètre du faisceau laser : 2800 µm ;
- figure 7F : *Enterobacter Chloacae* - diamètre du faisceau laser : 2800 µm.

Les figures 8A à 8D montrent des observations de colonies de différentes tailles. La figure 8A montre une observation au microscope d'une microcolonie de *Staphylococcus epidermidis* de diamètre 760 µm. La figure 8B représente un diffractogramme de cette microcolonie. La figure 8C montre une observation au microscope d'une microcolonie *d'Escherichia coli* de diamètre 1160 µm. La figure 8D représente un diffractogramme de cette microcolonie.

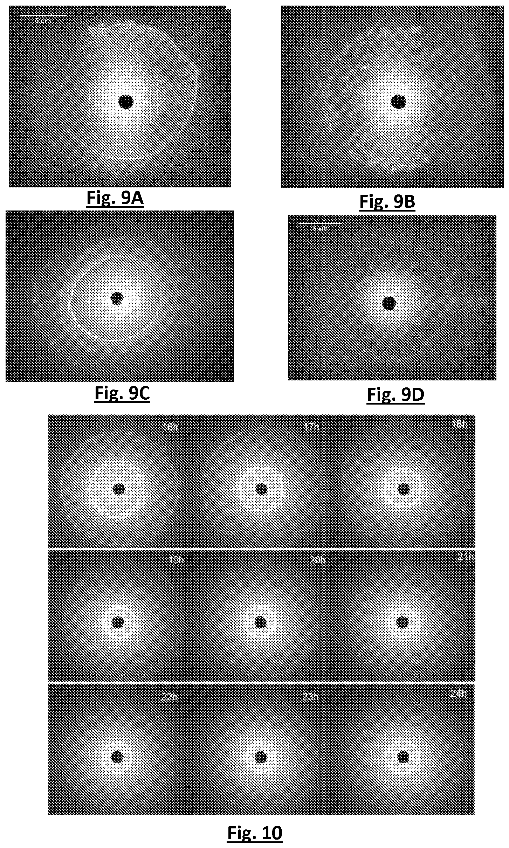

Les figures 9A à 9C montrent des diffractogrammes obtenus sur différentes géloses :
- la figure 9A représente un diffractogramme de *Staphylococcus saprophyticus* sur une gélose chocolat Polyvitex (PVX) ;
- la figure 9B représente un diffractogramme de *Pseudomonas putida* sur une gélose Mueller Hinton ;
- La figure 9C représente un diffractogramme *d'Escherichia coli* sur une gélose trypto-caséine soja (TSA).

Ces figures illustrent la compatibilité de l'invention avec différents milieux de culture 4, qu'ils soient opaques (COS, PVX) ou transparents (TSA).

La figure 9D représente un diffractogramme d'une colonie de *Staphylococcus saprophyticus* se développant sur une surface formée par un tapis de *Pseudomonas putida.* Ce résultat montre que l'invention permet une observation non destructive d'une colonie in-situ, sans avoir à effectuer un prélèvement.

Le procédé d'observation de l'invention est non destructif et peut être directement appliqué sur une colonie, dans son milieu de culture. Cela permet d'observer une cinétique du développement d'une colonie. La figure 10 représente une évolution temporelle d'un diffractogramme d'une même colonie de *Staphylococcus epidermidis* sur un milieu COS. Chaque vignette de cette figure est un diffractogramme de la colonie, l'intervalle temporel entre deux vignettes successives étant de 1h. La première vignette (en haut à gauche) correspond à une durée d'incubation de 16h, la dernière vignette (en bas à droite) correspondant à une durée d'incubation de 24h. La durée d'incubation est mentionnée en haut à droite de chaque vignette.

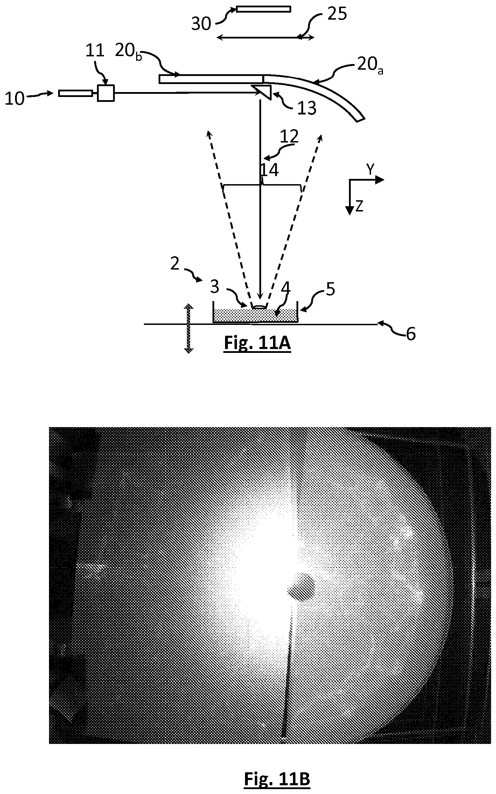

La figure 11A représente un dispositif expérimental utilisé au cours d'un essai de façon à comparer une image obtenue avec un écran plat avec une image obtenue avec un écran en forme de dôme, tel que décrit en lien avec la figure 3E. L'écran 20 comporte une partie incurvée 20a et une partie plane 20b.La partie incurvée 20a est une partie d'un hémisphère de verre poli. La partie plane 20b est formée par un papier calque. Un essai a été réalisé, en utilisant une colonie bactérienne *Pseudomonas putida,* sur une gélose TSA. Le diffractogramme obtenu est représenté sur la figure 11B. On observe que le diffractogramme déborde du calque plan, mais est contenu sur le dôme.

L'invention pourra être mise en œuvre en support à différents types d'examens, de type test de stérilité, test de susceptibilité à des antibiotiques, test de susceptibilités à des antibactériens ou aux bactériophages, criblage de substances antibactériennes, identification, dénombrement. L'invention peut également s'appliquer à l'observation et à la caractérisation d'autres types de microorganismes, comme des levures, des champignons ou des microalgues.

## Revendications

1. Dispositif pour observer un objet (3), présent dans un échantillon (2) comportant :
- un support (6), apte à recevoir l'échantillon (2), le support définissant un plan (XY), dit plan d'échantillon, selon lequel s'étend l'échantillon lorsqu'il est disposé sur le support;
- une source de lumière (10), apte à émettre un faisceau lumineux (12), dit faisceau lumineux incident, pour illuminer l'objet ;
- un capteur d'image (30), pour acquérir une image représentative d'un rayonnement (14) rétrodiffusé par l'objet sous l'effet d'une illumination par le faisceau lumineux incident (12) ;
- un écran (20), s'étendant en regard du support (6), de manière à être exposé à un rayonnement rétrodiffusé par l'objet lorsque ce dernier est illuminé par le faisceau lumineux incident, de façon à former, sur l'écran, une image (I₂₀), dite diffractogramme, représentative dudit rayonnement rétrodiffusé ;
- l'écran comportant une première face (20₁) exposée au rayonnement rétrodiffusé, l'aire de la première face étant supérieure à 100 cm² ;
- le capteur d'image (30) étant configuré pour acquérir une image (l₃₀) du diffractogramme formé sur l'écran ;
le dispositif comportant également un élément réfléchissant (13), disposé entre l'écran (20) et l'objet (3), apte à réfléchir une partie du faisceau lumineux incident (12) selon un axe d'incidence (Z) perpendiculaire ou sensiblement perpendiculaire au plan de l'échantillon (XY), l'élément réfléchissant étant solidaire de la première face (20₁) de l'écran ;
le dispositif étant **caractérisé en ce qu'**il comporte un support de liaison (17) s'étendant entre l'élément réfléchissant (13) et l'écran (20), le support de liaison permettant une fixation de l'élément réfléchissant (13) sur l'écran (20), le dispositif étant tel que l'élément réfléchissant (13) et/ou le support de liaison (17) sont configurés pour absorber au moins 50% du rayonnement rétrodiffusé (14) se propageant entre l'objet (3) et l'écran (20).

2. Dispositif selon la revendication 1, dans lequel l'écran (20) est incurvé.

3. Dispositif selon l'une quelconque des revendications précédentes, dans lequel :
- l'écran (20) comporte une deuxième face (20₂), de façon que le diffractogramme formé sur la première face (20₁) apparaisse sur la deuxième face ;
- l'écran s'étend entre le capteur d'image (30) et le support (6), de telle sorte que le capteur d'image (30) est couplé à la deuxième face (20₂) par une optique de focalisation (25).

4. Dispositif selon l'une quelconque des revendications précédentes, dans lequel la distance (δ) entre l'élément réfléchissant (13) et l'écran (20) est inférieure à 2 cm.

5. Dispositif selon l'une quelconque des revendications précédentes, dans lequel la surface de l'élément réfléchissant est inférieure à 4 cm² ou à 2 cm² ou à 1 cm².

6. Dispositif selon l'une quelconque des revendications précédentes, dans lequel l'écran (20) est translucide.

7. Dispositif selon l'une quelconque des revendications 3 à 6, dans lequel l'écran (20) comporte un guide de lumière pour transporter une lumière entre la première face (20₁) et la deuxième face (20₂).

8. Dispositif selon la revendication 7, dans lequel l'écran comporte plusieurs fibres optiques s'étendant entre la première face et la deuxième face.

9. Dispositif selon l'une quelconque des revendications 1 ou 4 ou 5, dans lequel l'écran (20) est une partie photosensible du capteur d'image (30).

10. Dispositif selon l'une quelconque des revendications précédentes, dans lequel l'écran (20) est mobile par rapport au support (6), la distance (d) entre le support et l'écran pouvant être ajustée.

11. Dispositif selon l'une quelconque des revendications précédentes, dans lequel le faisceau lumineux incident (12) se propage entre l'élément réfléchissant (13) et l'objet (10) selon un axe dit axe d'incidence, le dispositif comportant un réflecteur dit annulaire (18), s'étendant autour de l'axe d'incidence, entre l'échantillon (2) et l'écran (20), le réflecteur étant apte à réfléchir une partie du rayonnement rétrodiffusé (14) vers l'écran (20).

12. Procédé d'observation d'un objet (3) présent dans un échantillon (2), l'échantillon s'étendant face à un écran (20) comportant une première face (20₁), le procédé comportant les étapes suivantes :
a) illumination de l'objet (3) à l'aide d'un faisceau lumineux incident (12) émis par une source de lumière (10), le faisceau lumineux incident (12) se propageant jusqu'à un élément réfléchissant (13), disposé entre l'écran et l'objet, l'élément réfléchissant dirigeant tout ou partie du faisceau lumineux incident vers l'objet, l'élément réfléchissant étant relié à la première face de l'écran (20₁) ;
b) exposition d'une première face (20₁) d'un écran à un rayonnement lumineux (14) rétrodiffusé par l'échantillon sous l'effet de l'illumination, de façon à former, sur ladite première face, une image, dite diffractogramme, représentative dudit rayonnement rétrodiffusé (14), l'aire de ladite première face (20₁) étant supérieure à 100 cm²;
c) acquisition d'une image du diffractogramme formé sur l'écran par un capteur d'image (30) ;
le procédé étant **caractérisé en ce que** l'élément réfléchissant (13) est fixé sur l'écran (20) à travers un support de liaison (17) s'étendant entre l'élément réfléchissant (13) et l'écran (20), le rayonnement incident (12) se propage de l'élément réfléchissant (13) jusqu'à l'objet (10) selon un axe d'incidence, et **en ce qu'**au moins 50% du rayonnement rétrodiffusé (14) se propageant vers l'écran, parallèlement à l'axe d'incidence, est absorbé avant d'atteindre l'écran, par l'élément réfléchissant (13) ou par le support de liaison (17), de façon à former une ombre sur le diffractogramme formé sur l'écran.

13. Procédé selon la revendication 12, comportant, suite à l'étape c), une étape d'ajustement de la distance (d) entre l'échantillon (2) et l'écran (20) en fonction de l'image acquise par le capteur d'image (30), les étapes a) à c) étant répétées après l'ajustement de ladite distance.

14. Procédé selon l'une quelconque des revendications 12 ou 13, comportant une étape d) de caractérisation de l'objet (3) à partir de l'image acquise par le capteur d'image.

15. Procédé selon l'une quelconque des revendications 12 à 14, dans lequel l'objet comporte un microorganisme.

## Patentansprüche

1. Vorrichtung zur Beobachtung eines Objekts (3), das in einer Probe (2) vorhanden ist, umfassend:
- einen Träger (6), der dazu fähig ist, die Probe (2) aufzunehmen, wobei der Träger eine Ebene (XY) definiert, die als Probenebene bezeichnet wird und gemäß derer sich die Probe erstreckt, wenn sie auf dem Träger angeordnet ist;
- eine Lichtquelle (10), die dazu fähig ist, ein Lichtbündel (12), das als einfallendes Lichtbündel bezeichnet wird, zu emittieren, um das Objekt zu beleuchten;
- einen Bildsensor (30), um ein Bild zu erfassen, das für eine Strahlung (14), die unter der Einwirkung einer Beleuchtung durch das einfallende Lichtbündel (12) von dem Objekt rückgestreut wird, repräsentativ ist;
- einen Schirm (20), der sich so gegenüber dem Träger (6) erstreckt, dass er einer von dem Objekt rückgestreuten Strahlung ausgesetzt ist, wenn dieses durch das einfallende Lichtbündel beleuchtet wird, sodass auf dem Schirm ein Bild (I₂₀), das als Diffraktogramm bezeichnet wird, gebildet wird, welches für die rückgestreute Strahlung repräsentativ ist;
- wobei der Schirm eine erste Seite (20₁) umfasst, die der rückgestreuten Strahlung ausgesetzt ist, wobei die Fläche der ersten Seite größer als 100 cm² ist;
- wobei der Bildsensor (30) dazu konfiguriert ist, ein Bild (I₃₀) des auf dem Schirm gebildeten Diffraktogramms zu erfassen;
wobei die Vorrichtung ferner ein reflektierendes Element (13) umfasst, das zwischen dem Schirm (20) und dem Objekt (3) angeordnet ist und dazu fähig ist, einen Teil des einfallenden Lichtbündels (12) gemäß einer Einfallsachse (Z), die zu der Probenebene (XY) senkrecht oder im Wesentlichen senkrecht ist, zu reflektieren, wobei das reflektierende Element fest an der ersten Seite (20₁) des Schirms angebracht ist; wobei die Vorrichtung **dadurch gekennzeichnet ist, dass** sie einen Verbindungsträger (17) umfasst, der sich zwischen dem reflektierenden Element (13) und dem Schirm (20) erstreckt, wobei der Verbindungsträger eine Befestigung des reflektierenden Elements (13) an dem Schirm (20) gestattet, wobei die Vorrichtung derart ist, dass das reflektierende Element (13) und/oder der Verbindungsträger (17) dazu konfiguriert sind, mindestens 50 % der rückgestreuten Strahlung (14), die sich zwischen dem Objekt (3) und dem Schirm (20) ausbreitet, zu absorbieren.

2. Vorrichtung nach Anspruch 1, wobei der Schirm (20) gekrümmt ist.

3. Vorrichtung nach einem der vorhergehenden Ansprüche, wobei:
- der Schirm (20) eine zweite Seite (20₂) umfasst, sodass das auf der ersten Seite (20₁) gebildete Diffraktogramm auf der zweiten Seite erscheint;
- sich der Schirm so zwischen dem Bildsensor (30) und dem Träger (6) erstreckt, dass der Bildsensor (30) durch eine Fokussierungsoptik (25) mit der zweiten Seite (20₂) gekoppelt ist.

4. Vorrichtung nach einem der vorhergehenden Ansprüche, wobei der Abstand (δ) zwischen dem reflektierenden Element (13) und dem Schirm (20) weniger als 2 cm beträgt.

5. Vorrichtung nach einem der vorhergehenden Ansprüche, wobei die Oberfläche des reflektierenden Elements weniger als 4 cm² oder 2 cm² oder 1 cm² beträgt.

6. Vorrichtung nach einem der vorhergehenden Ansprüche, wobei der Schirm (20) transluzent ist.

7. Vorrichtung nach einem der Ansprüche 3 bis 6, wobei der Schirm (20) einen Lichtleiter umfasst, um ein Licht zwischen der ersten Seite (20₁) und der zweiten Seite (20₂) zu übertragen.

8. Vorrichtung nach Anspruch 7, wobei der Schirm mehrere optische Fasern umfasst, die sich zwischen der ersten Seite und der zweiten Seite erstrecken.

9. Vorrichtung nach einem der Ansprüche 1 oder 4 oder 5, wobei der Schirm (20) ein lichtempfindlicher Teil des Bildsensors (30) ist.

10. Vorrichtung nach einem der vorhergehenden Ansprüche, wobei der Schirm (20) mit Bezug auf den Träger (6) beweglich ist, wobei der Abstand (d) zwischen dem Träger und dem Schirm einstellbar ist.

11. Vorrichtung nach einem der vorhergehenden Ansprüche, wobei sich das einfallende Lichtbündel (12) zwischen dem reflektierenden Element (13) und dem Objekt (10) gemäß einer Achse, die als Einfallsachse bezeichnet wird, ausbreitet, wobei die Vorrichtung einen als ringförmig bezeichneten Reflektor (18) umfasst, der sich zwischen der Probe (2) und dem Schirm (20) um die Einfallsachse erstreckt, wobei der Reflektor dazu fähig ist, einen Teil der rückgestreuten Strahlung (14) zu dem Schirm (20) zu reflektieren.

12. Verfahren zur Beobachtung eines Objekts (3), das in einer Probe (2) vorhanden ist, wobei sich die Probe gegenüber einem Schirm (20) erstreckt, der eine erste Seite (20₁) umfasst, wobei das Verfahren die folgenden Schritte umfasst:
a) Beleuchten des Objekts (3) mit Hilfe eines einfallenden Lichtbündels (12), das von einer Lichtquelle (10) emittiert wird, wobei sich das einfallende Lichtbündel (12) bis zu einem reflektierenden Element (13), das zwischen dem Schirm und dem Objekt angeordnet ist, ausbreitet, wobei das reflektierende Element das gesamte oder einen Teil des einfallenden Lichtbündels zu dem Objekt lenkt, wobei das reflektierende Element mit der ersten Seite des Schirms (20₁) verbunden ist;
b) Aussetzen einer ersten Seite (20₁) eines Schirms gegenüber einer Lichtstrahlung (14), die unter der Einwirkung der Beleuchtung von der Probe rückgestreut wird, um auf der ersten Seite ein Bild, das als Diffraktogramm bezeichnet wird, zu bilden, welches für die rückgestreute Strahlung (14) repräsentativ ist, wobei die Fläche der ersten Seite (20₁) größer als 100 cm² ist;
c) Erfassen eines Bildes des auf dem Schirm gebildeten Diffraktogramms durch einen Bildsensor (30);
wobei das Verfahren **dadurch gekennzeichnet ist, dass** das reflektierende Element (13) mittels eines Verbindungsträgers (17), der sich zwischen dem reflektierenden Element (13) und dem Schirm (20) erstreckt, an dem Schirm (20) befestigt ist, dass sich die einfallende Strahlung (12) gemäß einer Einfallsachse von dem reflektierenden Element (13) bis zu dem Objekt (10) ausbreitet und dadurch, dass mindestens 50 % der rückgestreuten Strahlung (14), die sich parallel zu der Einfallsachse zu dem Schirm ausbreitet, vor dem Erreichen des Schirms von dem reflektierenden Element (13) oder von dem Verbindungsträger (17) absorbiert werden, sodass auf dem auf dem Schirm gebildeten Diffraktogramm ein Schatten gebildet wird.

13. Verfahren nach Anspruch 12, das im Anschluss an den Schritt c) einen Schritt des Einstellens des Abstands (d) zwischen der Probe (2) und dem Schirm (20) in Abhängigkeit von dem durch den Bildsensor (30) erfassten Bild umfasst, wobei die Schritte a) bis c) nach dem Einstellen des Abstands wiederholt werden.

14. Verfahren nach einem der Ansprüche 12 oder 13, das einen Schritt d) der Charakterisierung des Objekts (3) anhand des durch den Bildsensor erfassten Bildes umfasst.

15. Verfahren nach einem der Ansprüche 12 bis 14, wobei das Objekt einen Mikroorganismus umfasst.

## Claims

1. Device for observing an object (3), present in a sample (2), comprising:
- a holder (6), able to receive the sample (2), the holder defining a plane (XY), called the sample plane, in which the sample lies when it is placed on the holder;
- a light source (10), able to emit a light beam (12), called the incident light beam, in order to illuminate the object;
- an image sensor (30), for acquiring an image representative of radiation (14) back scattered by the object under the effect of an illumination by the incident light beam (12);
- a screen (20), lying facing the holder (6), so as to be exposed to radiation back scattered by the object when the latter is illuminated by the incident light beam, so as to form, on the screen, an image (I₂₀), called a scattergram, representative of said back-scattered radiation;
- the screen comprising a first face (20₁) exposed to the back-scattered radiation, the area of the first face being larger than 100 cm²;
- the image sensor (30) being configured to acquire an image (I₃₀) of the scattergram formed on the screen;
the device also comprising a reflective element (13), which is placed between the screen (20) and the object (3), and which is able to reflect a portion of the incident light beam (12) along an axis of incidence (Z) perpendicular or substantially perpendicular to the plane of the sample (XY), the reflective element being securely fastened to the first face (20₁) of the screen;
the device being **characterized in that** it comprises a binding medium (17) lying between the reflective element (13) and the screen (20), the binding medium allowing the reflective element (13) to be fastened to the screen (20), the device being such that the reflective element (13) and/or the binding medium (17) are configured to absorb at least 50% of the back-scattered radiation (14) propagating between the object (3) and the screen (20).

2. Device according to Claim 1, wherein the screen (20) is curved.

3. Device according to any one of the preceding claims, wherein:
- the screen (20) comprises a second face (20₂), so that the scattergram formed on the first face (20₁) appears on the second face;
- the screen lies between the image sensor (30) and the holder (6), in such a way that the image sensor (30) is coupled to the second face (20₂) by a focusing optic (25).

4. Device according to any one of the preceding claims, wherein the distance (δ) between the reflective element (13) and the screen (20) is smaller than 2 cm.

5. Device according to any one of the preceding claims, wherein the area of the reflective element is smaller than 4 cm² or than 2 cm² or than 1 cm².

6. Device according to any one of the preceding claims, wherein the screen (20) is translucent.

7. Device according to any one of Claims 3 to 6, wherein the screen (20) comprises a light guide for conveying light between the first face (20₁) and the second face (20₂).

8. Device according to Claim 7, wherein the screen comprises a plurality of optical fibres extending between the first face and the second face.

9. Device according to any one of Claims 1 or 4 or 5, wherein the screen (20) is a photosensitive portion of the image sensor (30).

10. Device according to any one of the preceding claims, wherein the screen (20) is movable with respect to the holder (6), the distance (d) between the holder and the screen being adjustable.

11. Device according to any one of the preceding claims, wherein the incident light beam (12) propagates between the reflective element (13) and the object (10) along an axis called the axis of incidence, the device comprising what is called an annular reflector (18), lying around the axis of incidence, between the sample (2) and the screen (20), the reflector being able to reflect some of the radiation (14) back scattered toward the screen (20).

12. Method for observing an object (3) present in a sample (2), the sample lying facing a screen (20) comprising a first face (20₁), the method comprising the following steps:
a) illuminating the object (3) using an incident light beam (12) emitted by a light source (10), the incident light beam (12) propagating to a reflective element (13) placed between the screen and the object, the reflective element directing all or some of the incident light beam toward the object, the reflective element being joined to the first face of the screen (20₁);
b) exposing a first face (20₁) of a screen to light radiation (14) back scattered by the sample under the effect of the illumination, so as to form, on said first face, an image, called a scattergram, representative of said back-scattered radiation (14), the area of said first face (20₁) being larger than 100 cm²;
c) acquiring an image of the scattergram formed on the screen with an image sensor (30);
the method being **characterized in that** the reflective element (13) is fastened to the screen (20) by a binding medium (17) lying between the reflective element (13) and the screen (20), the incident radiation (12) propagates from the reflective element (13) to the object (10) along an axis of incidence, and **in that** at least 50% of the back-scattered radiation (14) propagating toward the screen, parallel to the axis of incidence, is absorbed before reaching the screen, by the reflective element (13) or by the binding medium (17), so as to form a shadow in the scattergram formed on the screen.

13. Method according to Claim 12, comprising, following step c), a step of adjusting the distance (d) between the sample (2) and the screen (20) depending on the image acquired by the image sensor (30), steps a) to c) being repeated after the adjustment of said distance.

14. Method according to either one of Claims 12 and 13, comprising a step d) of characterizing the object (3) on the basis of the image acquired by the image sensor.

15. Method according to any one of Claims 12 to 14, wherein the object comprises a microorganism.
